# EUROPÄISCHE PATENTSCHRIFT

(11) **EP 0 517 030 B1**
(45) Veröffentlichungstag und Bekanntmachung des Hinweises auf die Patenterteilung: **04.09.1996**
(21) Anmeldenummer: 92108405.9
(22) Anmeldetag: 19.05.1992
(51) Int. Cl.: A61F 2/44

(54) **Wirbelkörperimplantat**
Vertebral implant
Implant vertébral

(30) Priorität: 04.06.1991 DE 4118316; 08.05.1992 DE 4215137
(43) Veröffentlichungstag der Anmeldung: 09.12.1992
(73) Patentinhaber: MAN Ceramics GmbH, 94453 Deggendorf (DE)
(72) Erfinder: Siebels, Wolfgang, W-8360 Deggendorf (DE); Ascherl, Rudolf, Dr., W-8000 München 40 (DE)

(56) Entgegenhaltungen:
- EP-A- 0 197 441
- EP-A- 0 302 719
- EP-A- 0 307 241
- WO-A-88/05312
- WO-A-90/00037
- DE-A- 2 426 814
- DE-A- 3 023 942
- US-A- 3 867 728
- US-A- 4 743 256

## Beschreibung

Die Erfindung bezieht sich auf ein Implantat für Wirbelsäulen, das direkt zwischen zwei angrenzenden wirbelkörpern zwischenlegbar ist.

Wirbelkörper haben entlang einer Wirbelsäule unterschiedliche Größen und sind von Patient zu Patient auch unterschiedlich. Beim Ersetzen eines Wirbelkörpers durch ein Implantat ist es daher notwendig, das Implantat an die effektive Dimension des Abstandes zwischen den angrenzenden Wirbelkörpern anzupassen.

Um diesen Umstand Rechnung zu tragen, wurden Implantate entwickelt (DE 30 23 942 C3), die aus im wesentlichen zwei in Schraubenverbindung stehenden Teilen bestehen, und deren axiale Höhe durch Drehen verändert bzw. an den Abstand zwischen den Wirbelkörpern angepaßt werden kann. Mittels Querschrauben oder anderen Verankerungsmitteln werden die beiden Teile nach ihrer Einstellung drehsicher verankert. Damit läßt sich zwar mittels einer Ausführung eine ganze Bandbreite von Abständen abdecken, aber die Höheneinstellung nimmtjedoch insbesondere bei einem feinen Gewinde relativ viel Zeit in Anspruch.

Diesen Nachteil behebend, ist aus der WO 90/00037 ein Implantat der gattungsgemäßen Art bekanntgeworden. das mittels eines Werkzeugs lediglich zwischen zwei Wirbel eingeschoben wird. Das annähernd quaderförmige Implantat wird jedoch aus komplizierten Einzelteilen zusammengesetzt.

Der Erfindung liegt daher die Aufgabe zugrunde, ein Implantat der eingangs genannten Art zu entwickeln, das rasch implantierbar ist, aber auch fertigungstechnisch einfach für eine Vielfalt von Abmessungen hergestellt werden kann.

Die Aufgabe wird erfindungsgemäß mit den Merkmalen des Anspruches 1 gelöst.

Gemäß der Erfindung werden zur Bildung eines Wirbelkörperimplantats zwei oder mehrere Scheiben zusammengesetzt. Hierbei wird ein Vorrat von einem Sortiment von Scheiben unterschiedlicher Höhen und Durchmesser gehalten. Für eine Implantation wird der Abstand zwischen den Wirbeln gemessen und entsprechend dicke bzw. hohe Scheiben aus dem Sortiment zusammen kombiniert, derart, daß sie in ihrer Gesamtheit die gewünschte Höhenabmessung haben. Die herausgesuchten Scheiben, sie bestehen aus Teilen gleicher Form, nur mit unterschiedlicher Höhe, werden im Baukastenprinzip aufein andergestapelt und als fertiges Implantat zwischen die Wirbelkörper gesetzt, die dazu geringfügig auseinandergezogen werden. Auch hier ist nach dem Einsetzen des Implantats keine Regulierung oder Justierung desselben innerhalb des Patientenkörpers erforderlich.

Mit Hilfe eines Computers lassen sich die zu kombinierenden Höhen der Scheiben sekundenschnell ermitteln, so daß zwischen Wirbelabstands-Vermessung bis zum Erhalt des einsetzbaren Implantats ein minmaler Zeitaufwand erforderlich ist. Die radiale und drehsichere Verankerung der zusammengesetzten Scheiben läßt sich vielfältig bewältigen.

Gemäß einer Ausgestaltung der Erfindung weisen die Scheiben fluchtende Bohrungen auf, in die Verankerungsstifte eingesetzt werden. Bei dieser Ausgestaltung sind die Scheiben sowohl radial als auch drehsicher miteinander verbunden. Außerdem sind die Scheiben fertigungstechnisch sehr einfach herzustellen.

Eine andere Möglichkeit ist, die Scheiben direkt mit eingeformten Verankerungsmitteln, wie z.B. Nut und Feder, Stift und Bohrung, herzustellen.

Auch die Scheibenpackungen können als Ringscheiben ausgebildet werden, wobei der Hohlraum zur radialen Verankerung der Ringe mit Knochenmaterial oder -zement ausgefüllt werden kann. Vorteilhaft ist es, wenn der Innenmantel der Ringscheiben unregelmäßig ist oder geometrische Unregelmäßigkeiten aufweist. jede Abweichung von der kreiszylindrischen Form dient zur drehsicheren Verankerung der aufgestapelten Scheiben, wenn der Hohlraum der Ringscheiben mit einem härtenden Material ausgefüllt wird.

Für den sicheren Halt des als Scheibenstapel ausgebildeten Implantats zwischen den angrenzenden Wirbelkörpern werden Endscheiben mit einer rauhen Stirnseite vorgesehen. Die Rauhigkeit kann durch eine strukturierte Oberfläche, herausragende Spitzen, Wellen und dergleichen erzeugt werden.

In jeder Ausführung ist es möglich, die Scheiben zu einer soliden Einheit miteinander zu verkleben, z.B. mit PMMA-Zement, wenn erforderlich oder zweckmäßig.

Die Scheiben werden vorzugsweise aus einem kohlenstoffaserverstärkten Kunststoff (CFK) hergestellt, wobei die Verankerungsmittel je nach Ausgestaltung des Implantats aus demselben oder einem anderen Material bestehen können. Die Herstellung des gesamten Implantats aus CFK hat den Vorteil, daß das Implantat keine Streuung von Strahlen bewirkt, so daß die Wirbelsäule und das angrenzende biologische Gewebe auch nach dem Implantieren eines Wirbelkörperersatzes mit allen bildgebenden Verfahren (CT, MR) untersucht werden kann

Bekannte Wickeltechniken lassen sich zur serienmäßigen Fertigung der Implantat-Elemente anwenden. Die Ringscheiben können beispielsweise mittels einer Flechtmaschine, die zusätzlich mit unidirektionalen Fasern (UD) bestückt ist, hergestellt werden. Mittels eines Stabdornes, der durch das Flechtauge gezogen und mit UD-Fasern und Flechtwerk umlegt wird, wird ein Faserverbundrohr in einem Arbeitsgang hergestellt, von dem dann die Ringscheiben abgeschnitten werden. Der Stabdorn ist vorzugsweise aus dem auch als Trennmittel verwendbaren PTFE (Polytetrafluorethylen). Der Stabdorn kann dabei ein Vieleck als Querschnitt haben oder über die Länge Nuten und/oder Erhebungen aufweisen, wodurch im Faserverbundrohr bzw. in den Ringscheiben die für die drehsichere Verankerung dieerforderliche Innenmantelgeometrie direkt bei deren Herstellung gebildet wird.

Auch Wickelverfahren unter Anwendung von Fasern oder Fasergelegen erlauben fertigungstechnisch einfache und für Serienfertigung geeignete Herstellverfahren. Es können einheitliche Streben für die Einzelscheiben und die Scheibenpackungen konzipiert werden.

Die Erfindung wird anhand von in der Zeichnung schematisch dargestellten Ausführungsbeispielen näher erläutert. Es zeigen:
- Figuren 1 und 2: ein erstes Ausführungsbeispiel,
- Figuren 3 und 4: ein zweites Ausführungsbeispiel,
- Figuren 5 bis 8: je ein weiteres Ausführungsbeispiel.

Der Erfindung liegt der Gedanke zugrunde, daß der Chirurg an Ort und Stelle direkt nach Kenntnis der tatsächlichen Abmessungen den Wirbelkörperersatz zusammenstellt, ohne die Hilfe eines Prothesentechnikers. Dazu wird ein Vorrat von Implantatkomponenten unterschiedlicher Durchmesser und Höhen gehalten, so daß für den jeweiligen Fall die entsprechende Anzahl von Komponenten mit entsprechenden Abmessungen herausgeholt und zusammengesetzt zu werden braucht, ohne Schraubjustier-oder andere Handgriffe vornehmen zu müssen. Die Auswahl der Scheiben nach ihrer Höhe im letzten Fall kann mittels eines Rechners erfolgen.

Die Grundlage eines zusammengesetzten Implantats besteht im Aufstapeln von vorgefertigten Scheiben, wobei diese Scheiben eine runde, mehreckige oder unregelmäßige Außenkontur haben können. Es können volle Scheiben oder auch Ringscheiben als Komponenten verwendet werden. Es werden Scheibensätze mit unterschiedlichen Durchmessern benötigt, wobei jeder Satz eines Durchmessers mit Scheiben unterschiedlicher Höhe bestückt ist. Steht der Durchmesser des einzusetzenden Implantats fest, so werden in dem entsprechenden Scheibensatz noch die entsprechenden Höhen ausgesucht, so daß nach dem Zusammensetzen der gewählten Scheiben sich die erforderliche Implantathöhe ergibt.

Um das Sortiment bezüglich der Scheibenhöhe möglichst klein zu halten, können beispielsweise wenige hohe Abmessungen vorgesehen werden, die mit niedrigen Scheiben, z.B. millimeterdicken Scheiben, entsprechend ergänzt werden.

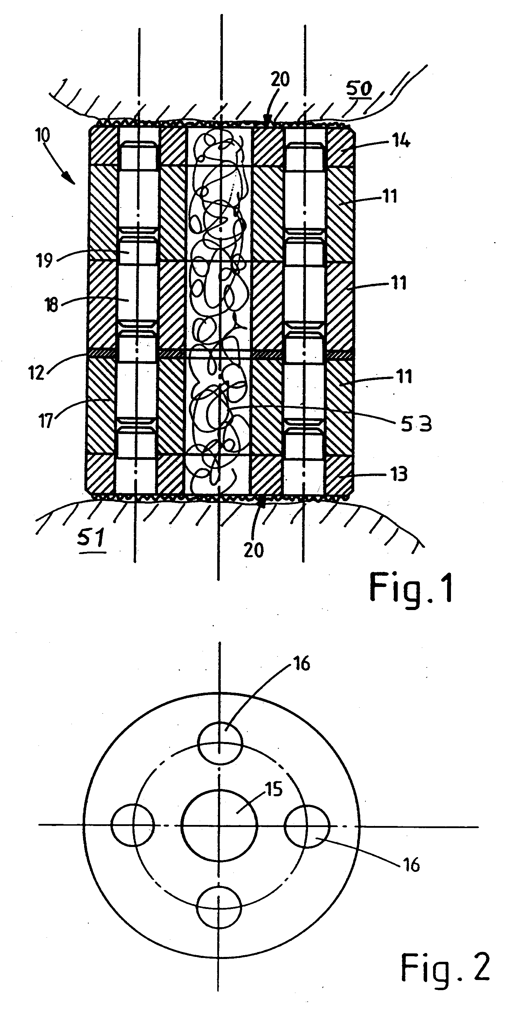

In Fig. 1 ist ein Ausführungsbeispiel gezeigt, bei dem ein fertiges Implantat 10 aus drei dickeren Scheiben 11, einer dünnen Scheibe 12 und zwei Endscheiben 13 bzw. 14 zusammengesetzt ist.

Wie in Fig. 2 dargestellt ist, bestehen die Scheiben 11 bis 14 aus runden Ringscheiben mit einer Innenbohrung 15 und jeweils vier regelmäßig auf die Ringscheibe verteilten Bohrungen 16. In diese Bohrungen 16 werden Verankerungsstifte 17 eingeführt. Gemaß der Ausführung nach Fig. 1 sind die Stifte 17 mit ihrem jeweils einem Ende 18 mit einer Scheibe 11, 13 verbunden, während sie mit dem anderen Ende 19 in die Bohrung einer nächsten Scheibe 11 hineinragen. Bei dieser Ausgestaltung wird eine Endscheibe 14 ohne Stift auszugestalten sein. In gleicher Weise werden die dünnen Scheiben 12 lediglich Bohrungen 16 aufweisen.

Es ist natürlich auch möglich, die Stifte 17 als von den Scheiben 11 bis 14 getrennte Bauteile herzustellen, so daß die Stifte erst bei dem Zusammensetzen eines Implantats 10 in die Bohrungen 16 eingeführt werden.

Anstelle von Stiften als Verankerungsmittel können auch Nut- und Federsysteme in jeder möglichen Konfiguration vorgesehen werden.

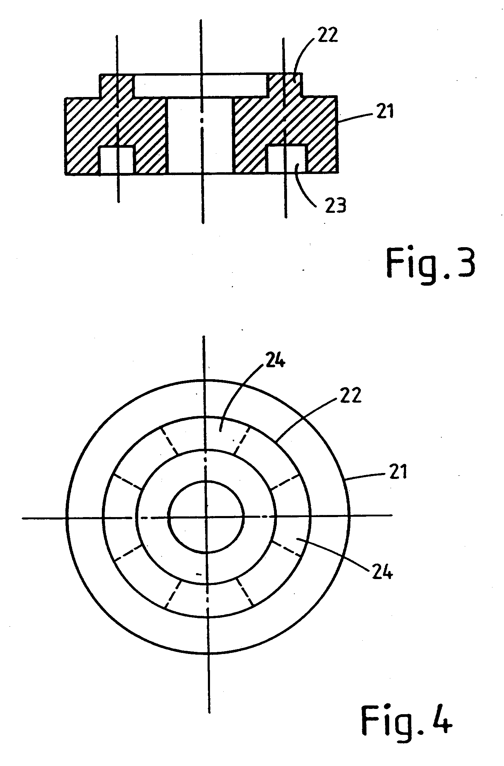

In Fig. 3 ist ein Ausführungsbeispiel gezeigt, bei dem die Scheiben 21 auf der einen Stirnseite mit einer Ringfeder 22 und auf der anderen Stirnseite mit einer damit fluchtenden Ringnut 23 versehen sind. Um eine Verankerung auch in Torsionsrichtung zu erreichen, können anstelle der Ringfeder 22, wie in Fig. 4 gestrichelt angedeutet, Federsegmente 24 vorgesehen werden, die in entsprechende Nutsegmente der nächsten Scheibe eingreifen.

In den dargestellten Ausführungsbeispielen wurden runde Scheiben mit kreissymmetrisch verteilten Verankerungselementen gezeigt. Es ist selbstverständlich jede asymmetrische Anordnung der Verankerungselemente sowie jede Außenkontur der Scheiben möglich, soweit letztere mit der Kontur der Wirbelkörper im Einklang steht

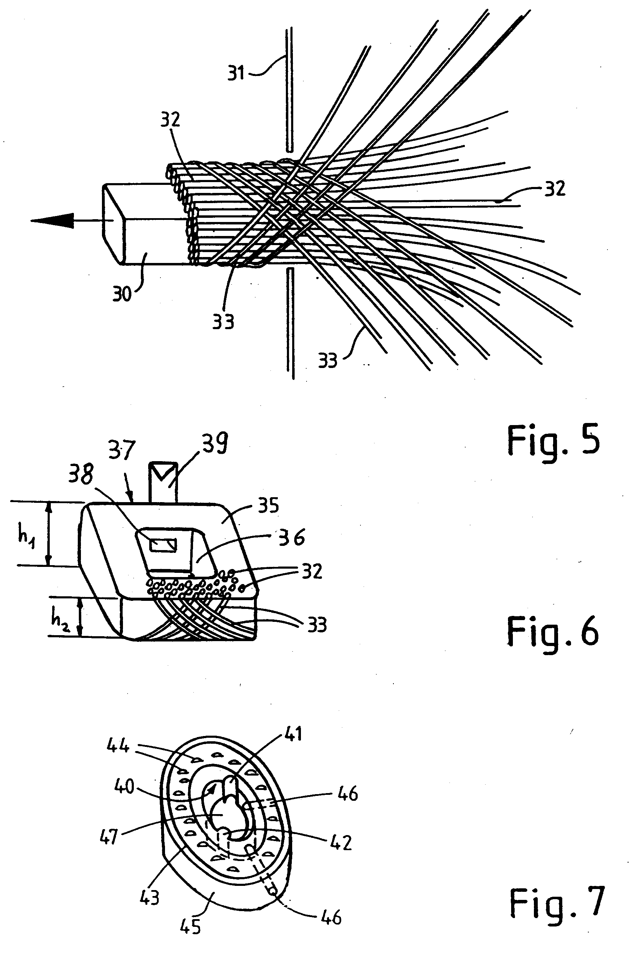

Ringscheiben oder volle Scheiben lassen sich fertigungstechnisch einfach aus jedem biokompatiblen Material herstellen, da sie an keine besondere Formgebung gebunden sind. Die Form kann sogar teilweise an das Herstellungsverfahren angepaßt werden. Für die Serienfertigung gut geeignete Herstellungsmethoden sind Wickeln oder Ziehen von Faserverbundrohren, aus denen die Scheiben als Einzelelement oder für die vorstehend beschriebenen Scheibenpackungen herausgesägt, -geschnitten bzw. -getrennt werden. Im Wickelverfahren können nach bekannten Methoden Fasern oder Fasermatten verwendet werden. Im Flechtverfahren wird, wie es in Fig. 5 angedeutet ist, ein entsprechend geformter Stabdorn 30, z.B. mit rechteckigem Querschnitt durch ein Fadenauge 31 durchgezogen und dabei mit Bündeln von längsgerichteten, mit Matrix imprägnierten UD-Fasern 32 sowie mit äußeren Flechtfasern 33 umgeben. Nach dem Aushärten der Matrix werden aus dem so hergestellten Faserverbundrohr Ringscheiben 35 herausgetrennt, wobei der Dorn vor oder nach der Trennung der Ringscheiben entfernt wird. Der als Faserverbundrohr ausgebildete Strang dient zur Herstellung von Scheiben für ein Scheibenpaket gemäß Fig. 1.

Einzelscheiben 35 werden bei Bedarf keilförmig (Fig. 6, h₁ > h₂) herausgetrennt. Im neutralen Bereich 37 können Öffnungen 38 vorgesehen werden, die zum Eingriff von Implantionswerkzeugen und Fixationsmitteln, wie Krampen 39 verwendet werden.

Der Hohlraum 36 kann mit fremdem oder dem patienteneigenen Knochenmaterial oder mit Knochenzement ausgefüllt werden, das ebenfalls durch die Öff nung 38 einführbar ist. Bei zusammengesetzten Scheiben dient der Knochenzement gleichzeitig zur Verankerung der Scheiben in radialer Richtung und aufgrund des nicht kreissymmetrischen Innenquerschnittes 36 auch in Torsionsrichtung. Anstelle des rechteckigen Innenquerschnittes kann jede andere Konfiguration außer der Kreisform zur Sicherung gegen Drehbeweglichkeit zwischen den Scheiben gewählt werden.

Fig. 7 zeigt eine Form mit einem zylindrischen Innenmantel 40, der mit einer Nut 41 und einer Erhebung 42 zur Torsionsverankerung ausgestattet ist.

Bei Bedarf werden die Scheiben oder Ringscheiben einseitig mit einer Klebstoffhülsen 44 einschließenden Starterfolie 43 versehen, wie in Fig. 7 gezeigt ist. Wenn zwei Scheiben 45 zur Bildung des Implantats aufeinandergelegt und axial gepreßt werden, platzen die Klebstoffhülsen 44 auf, so daß der Klebstoff sich zwischen den Scheiben 45 verteilt und die Scheiben miteinander verbindet. Die Klebverbindung kann als einzige Verbindung oder ergänzend zu den vorstehend genannten Verankerungsmitteln verwendet werden.

In der Ausführung nach Fig. 7 sind ferner Bohrungen 46 gezeigt, die radial durch die Ringscheibe 45 gefuhrt sind. Sie dienen zur Einführung des Knochenzements oder Knochenmaterials in den Hohlraum 47.

Die Endscheiben 13, 14 eines Implantats 10 haben an ihrem freien, die Auflage für den Wirbelknochen dienenden Stirnende eine rauhe, strukturierte oder mit diskreten Erhebungen versehene Oberfläche 20. Diese sollen in Zusammenwirkung mit den angrenzenden, gegen das Implantat 10 drückenden Wirbelkörpern 50, 51 die Verankerung innerhalb der Wirbelsäule gewährleisten und als Anwachshilfe dienen. Wie vorstehend beschrieben, kann bei Bedarf im implantierten Zustand durch eine nicht gezeigte radiale Bohrung Knochenzement oder - material 53 in die Innenbohrung 15 bis an die angrenzenden Wirbelkörper 50, 51 gedruckt werden. Eine rauhe Fläche läßt sich durch Verwendung einer grobkörnigen Schneidwerkzeuge direkt im Trennvorgang vom Strang bilden.

Die Implantation einesderartigen Wirbel- und/oder Bandscheibenersatzes bedingt keine systemspezifischen Schwierigkeiten. Wenn der chirurgische Schrittsoweit gekommen ist, daß der Abstand zwischen den angrenzenden Wirbelkörpern feststellbar ist, wird mittels dieses Wertes im Rechner die Zusammensetzung der Scheibenhöhen für das Implantat errechnet, herausgesucht und zusammengesetzt. Die angrenzenden Wirbelkörper werden etwas auseinandergezogen und das im Baukastensystem zusammengesetzte Implantat zwischengelegt. Außer dem Plazieren des Implantats sind keine weiteren Handgriffe bezüglich des Implantats notwendig. Außer der Implantathöhe variiert auch der Durchmesser des Implantats. Das Scheibensortiment ist daher auch nach Querschnitten zu bestücken.

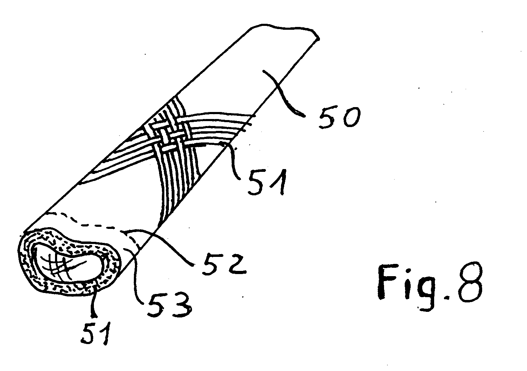

In Fig. 8 ist schließlich ein hohler Strang 50 unregelmäßiger Konfiguration gezeigt, der aus 1 bis 20 Flechtwerken 51 gebildet ist. Ein nicht gezeigter Dorn wird entsprechend oft durch das Ringfadenauge einer Flechtmaschine gezogen und dabei mit entsprechend vielen Flechtwerken und Matrixmaterial überzogen. Mit Trennscheiben werden an Trennlinien 52 die Scheiben 53 für ein Implantatelement herausgeschnitten.

## Patentansprüche

1. Implantat für die Wirbelsäule, das direkt zwischen zwei angrenzenden Wirbelkörpern (50) zwischenlegbar ist, dadurch gekennzeichnet, daß das Implantat (10) aus mindestens zwei aufeinander stapelbaren und radial miteinander verankerbaren steifen Scheiben (11 bis 14, 21, 35, 45, 53) gleicher oder unterschiedlicher Höhen besteht und daß die Auflageflächen (20) für die Wirbelkörper je nach Wirbellage parallel oder im Winkel zueinander stehen.

2. Implantat nach Anspruch 1, dadurch gekennzeichnet, daß die Scheiben als Ringscheiben (35, 45, 53) mit jeweils regelmäßigem oder unregelmäßigem Umfang ausgebildet sind.

3. Implantat nach Anspruch 1 oder 2, dadurch gekennzeichnet, daß die Auflageflächen (20) der beiden äußeren Scheiben (13,14) Rauhigkeiten, Porenwelligkeiten oder andere Unebenheiten in der Form von herausragenden Spitzen aufweisen.

4. Implantat nach einem der vorhergehenden Ansprüche, dadurch gekennzeichnet, daß die Scheiben (11 bis 14, 21, 35, 45, 53) aus faserverstärktem Kunststoff bestehen und im Wickelverfahren oder aus aufgerollten Fasermatten hergestellt sind und daß die Scheiben Kanäle (46) aufweisen, in die Knochenzement oder Knochenmaterial einbringbar ist.

5. Implantat nach einem der vorhergehenden Ansprüche, dadurch gekennzeichnet, daß die Scheiben aus einem Strang (32, 33 bzw. 50) geschnitten sind.

6. Implantat nach Anspruch 7, dadurch gekennzeichnet, daß der Strang (32,33 bzw. 50) aus unidirektionalen Fasern (32) und/oder aus Flechtlagen (33, 51) besteht.

7. Implantat nach einem der vorhergehenden Ansprüche, dadurch gekennzeichnet, daß die Scheiben (11 bis 14) fluchtende Bohrungen (16) aufweisen, in die für die radiale Verankerung Verankerungsstifte (19) einsetzbar sind.

8. Implantat nach einem der Ansprüche 1 bis 6, dadurch gekennzeichnet, daß die Scheiben (21) zur radialen Verankerung eingeformte Verankerungsmittel, wie Nut und Feder, Stift und Bohrung, aufweisen.

## Claims

1. An implant for the vertebral column directly arrangeable between two adjacent vertebral bodies (50), characterised in that the implant (10) comprises at least two rigid discs (11 to 14, 21, 35, 45, 53), fixable radially to each other and stackable on each other, of identical or different heights and, depending on the vertebral position, the bearing surfaces (20) for the vertebral bodies are parallel or at an angle to each other.

2. An implant according to Claim 1, characterised in that the discs are in the form of annular discs (35, 45, 53) with a respectively regular or irregular periphery.

3. An implant according to Claim 1 or 2, characterised in that the bearing surfaces (20) of the two outer discs (13, 14) have coarseness, pore ripples or other uneven features in the form of projecting peaks.

4. An implant according to any one of the preceding claims, characterised in that the discs (11 to 14, 21 35, 45, 53) consist of fibre-reinforced plastic and are produced using the rolling process or from rolled-up fibrous mats, and the discs have channels (46) into which bone cement or bone material may be inserted.

5. An implant according to any one of the preceding claims, characterised in that the discs are cut from a rope (32, 33 or 50 respectively).

6. An implant according to Claim 7 [sic - 5], characterised in that the rope (32, 33 or 50 respectively) consists of unidirectional fibres (32) and/or of braided layers (33, 51).

7. An implant according to any one of the preceding claims, characterised in that the discs (11 to 14) have aligned bores (16), and fixing pins (19) are insertable therein for radial fixing.

8. An implant according to any one of Claims 1 to 6, characterised in that the discs (21) have moulded fixing means for radial fixing, such as a tongue and groove, and a pin and bore.

## Revendications

1. Implant de colonne vertébrale, à intercaler directement entre deux vertèbres (50),
caractérisé en ce que
cet implant (10) est composé d'au moins deux disques rigides (11 à 14, 21, 35, 45, 53) de hauteurs égales ou non, empilables et ancrables radialement l'un sur l'autre, les portées d'appui (20) de l'implant sur les vertèbres étant parallèles ou faisant un angle entre elles, en fonction de la position des vertèbres.

2. Implant selon la revendication 1,
caractérisé en ce que
les disques sont des disques annulaires (35, 45, 53), à contour de forme régulière ou non.

3. Implant selon les revendications 1 ou 2,
caractérisé en ce que
les portées d'appui (20) des deux disques externes (13, 14) présentent des rugosités, des ondulations poreuses ou d'autres irrégularités en forme de pointes en relief.

4. Implant selon l'une des revendications précédentes,
caractérisé en ce que
les disques (11 à 14, 21, 35, 45, 53) sont en matière plastique renforcée par des fibres, obtenus par bobinage ou par enroulement de nattes de fibres, les disques étant percés de canaux (46) permettant d'introduire du ciment osseux ou de la matière osseuse.

5. Implant selon l'une des revendications précédentes,
caractérisé en ce que
les disques sont obtenus par tronçonnage d'une barre (32, 33 et 50).

6. Implant selon la revendication 5,
caractérisé en ce que
la barre (32, 33, 50) est constituée de fibres unidirectionnelles (32) et/ou de couches tressées (33, 51).

7. Implant selon l'une des revendications précédentes,
caractérisé en ce que
les disques (11 à 14) présentent des alésages (16) alignés dans lesquels peuvent être introduites des broches (19) assurant l'ancrage radial.

8. Implant selon l'une des revendications 1 à 6,
caractérisé en ce que
les disques (21) comportent des moyens d'ancrage radial par combinaison de formes, tels que rainure et languette, broche et alésage.
